(19) Europäisches Patentamt
European Patent Office
Office européen des brevets

(11) **EP 4 167 182 A1**

(12) **EUROPEAN PATENT APPLICATION**

(43) Date of publication:
**19.04.2023 Bulletin 2023/16**

(21) Application number: **21306439.7**

(22) Date of filing: **14.10.2021**

(51) International Patent Classification (IPC):
**G06T 7/00** (2017.01)

(52) Cooperative Patent Classification (CPC):
**G06T 7/0012;** G06T 2207/20084; G06T 2207/30088

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Assistance Publique Hôpitaux De Paris**
  **75004 Paris (FR)**
- **Sorbonne Université**
  **75006 Paris (FR)**

- **Université Paris-Saclay**
  **91190 Gif-sur-Yvette (FR)**

(72) Inventors:
- **AUREGAN, Jean-Charles**
  **92150 Suresnes (FR)**
- **BOSSER, Catherine**
  **69260 Charbonnières-les-Bains (FR)**
- **BACHY, Manon**
  **75012 Paris (FR)**

(74) Representative: **Cabinet Camus Lebkiri**
  **25 rue de Maubeuge**
  **75009 Paris (FR)**

(54) **METHOD FOR DETERMINING A CHARACTERISTIC LENGTH OF SKIN SURFACE REPRESENTATIVE OF A QUALITY OF COLLAGENIC INTERNAL ORGANS OF A PATIENT**

(57) According to an aspect of the invention, it is provided a non-invasive method (100) for determining a characteristic length representative of a quality of collagenic organs of a patient, characterized in that it comprises the following steps:
- Acquisition (110) of at least one image of a cutaneous replica of a portion of the skin surface of the patient, the cutaneous replica being obtained from a skin patching device (11) applied on the portion of the skin surface;

- Processing (120) of said acquired image in order to obtain a processed image, said processed image being formed by a plurality of geometrical shapes;
- Extraction (130) of a plurality of features from the plurality of geometrical shapes;
- Determination (140) of the characteristic length representative of the quality of the collagenic organs of the patient based on the extracted features

Fig. 2

## Description

## TECHNICAL FIELD

The technical field of the invention is the one of image processing and more specifically the one of image processing applied in a medical context.

The present invention concerns a method for determining a characteristic length of skin surface representative of a quality of collagenic internal organs of a patient. The present invention also relates to a system dedicated to the execution of the method.

## BACKGROUND OF THE INVENTION

A better quality of care provided to patients implies to better account for their clinical singularities and the associated specific needs. In particular, the question of ageing is complicated to deal with as the chronological age of a patient does not automatically reflect the actual biological age of his body, which is highly individual dependent.

In the case of collagenic organs (dermis, bones matrix tissues, dental matrix, arteries, abdominal wall, ligaments, tendons, etc.) their quality is strongly related to their actual biological age. In fact, the relative ageing appears to be the same for all internal and external collagenic organs, as the collagen concentration and its quality evolves the same way inside the whole body. Consequently, knowing the actual biological age of a collagenic organ would indicate the quality of all the other collagenic organs.

It is known that several parameters have an influence on the actual biological age and the quality of collagen organs, such as genetical endowment, chronic pathologies (e.g., diabetes, asthma, hypertension), living habits (e.g., tobacco, alcohol, sport), etc. The effect of those parameters on the ageing of a body can strongly change between individuals. As a consequence, depending on the quality of their respective collagenic organs, two individuals of the same chronological age would require different cares and treatments. Therefore, health professionals require biomarkers that are representative of the actual biological age of the patients collagenic organs in order to better select the therapeutic approach.

In the literature, two main biomarker types are used to determine the actual biological age of a patient's collagenic organs: blood senescence biomarkers and cutaneous biomarkers.

Blood senescence biomarkers, on one hand, are blood analysis driven biomarkers that are determined based on, for example, albumin rate, creatinine rate, glucose rate, C-reactive protein fraction, lymphocyte percent, mean cell volume, red blood cell distribution width, alkaline phosphatase rate, and white blood cell count. The obtained biomarkers are reliable and representative of the actual biological age of the patient organs (Liu et al., Phenotypic Age: a novel signature of mortality and morbidity risk, bioRxiv, 363291, 2018). However, such biomarkers require blood collection and analysis, which are not tractable during a medical consultation because the whole process requires a tedious invasive protocol and a dedicated equipment.

Cutaneous biomarkers, on the other hand, are more easily obtained as they are directly derived from skin associated data. The technique consists in analysing the skin properties of one individual to derive the actual biological age of collagenic organs. Several cutaneous biomarkers already exist, such as skin roughness, elastin to collagen ratio, microrelief profile, etc. Those biomarkers can be used to determine the quality of a collagenic organ (Shuster, Osteoporosis, a unitary hypothesis of collagen loss in skin and bone, Medical Hypotheses vol. 65(3), p. 426-432, 2005). However, the correlation between the quality and these cutaneous biomarkers is not sufficiently high to ensure a robust estimation of the actual biological age of collagenic organs based on these biomarkers. The error rate on the estimation of the quality of the collagenic organs is too high for the biomarkers to be reliable.

Therefore, there is a need for biomarkers that are robust to estimate the quality of internal collagenic organs of an individual, and that are easily and quickly tractable from simple non-invasive measurements.

In the prior art, the patent application WO2019/149393 discloses a method for evaluating the quality of one connective tissue in a patient by analysing the image of a cutaneous replica. The analysis is performed by an operator via a visual inspection on an image to determine the line shapes and anisotropy of the lines or with an optical sensor to determine the roughness index of the microrelief on an image. The analysis enables to identify a healthy skin or an altered skin and to relate to the health state of a dense connective tissue (e.g., bone tissues). Indeed, it is known that, on one hand, a young skin shows entangled collagenic fibres, a complex microstructure and a stretched skin, and on another hand, an aged skin shows hollowed out grooves, an altered or disappearing microstructure and a sagging skin. However, this method does not allow for a robust estimation of the internal collagenic organs quality as the analysis is operator dependent. The error rate is too high for the biomarkers to reliably estimate the quality the collagenic organs. Furthermore, as previously mentioned, this method does not also allow for a robust estimation as the chosen biomarkers and the quality of collagenic organs are not sufficiently correlated. Finally, the robustness of this technique is also penalized as this technique is not autonomous and is not compatible with a fast diagnostic (in the time of classical medical consultation).

## SUMMARY OF THE INVENTION

An object of the invention is to provide a solution to the aforementioned problems and limitations brought by the prior art by the implementation of a robust determination of a characteristic length, which is representative of the quality of collagenic organs, tractable from simple non-invasive measurements. The invention then allows for low error rate on the estimated collagenic organs quality.

More precisely, the invention discloses a method and a system to implement the method in order to determine the aforementioned characteristic length.

To this end, according to a first aspect of the invention, it is provided a non-invasive method for determining a characteristic length representative of a quality of collagenic organs of a patient, comprising the following steps:

- Acquisition of at least one image of a cutaneous replica of a portion of the skin surface of the patient, the cutaneous replica being obtained from a skin patching device applied on the portion of the skin surface;
- Processing of said acquired image in order to obtain a processed image, said processed image being formed by a plurality of geometrical shapes;
- Extraction of a plurality of features from the plurality of geometrical shapes;
- Determination of the characteristic length representative of the quality of the collagenic organs of the patient based on the extracted features.

Thanks to the invention, a practitioner has access to a characteristic length that is representative of a quality of collagenic organs. Indeed, the characteristic length is determined from at least one feature that is representative of a quality of the collagenic organs. On one hand, the characteristic length can be used by practitioners for assisting them in medical decision and prognosis. For example, the characteristic length can be used for monitoring the quality of a ligament after a sport trauma, to assist the design of a textile prosthesis in the case of an abdominal hernia, or to assist the prognosis of a genital prolapsus or a femoral neck fracture. Other examples concern the early detection and surveillance of systemic collagen pathologies such as osteoporosis and osteogenesis imperfecta classically called brittle bone disease due to a systemic alteration of collagen. On another hand, the characteristic length can be used for awareness-raising of patients in order, for example, to support them changing their life habits and increasing their well-being, in particular when the characteristic length reflects an actual biological age that is higher than the chronological age of the patient. The method can also be applied in a cosmetic context for testing new products.

Furthermore, the implementation of the method does not require any operator action. This makes the method automatic and less sensitive to measurement and analysis variability induced by operator actions. The characteristic length is then more reliable to assess the quality of collagenic organs with lower error rates than other techniques from the prior art. Another advantage is that the method is simple to implement.

Thanks to the characteristic length being representative of the quality of a patient collagenic organs and the method being operator independent, the method also allows for unified determination of the characteristic length. As a result, the method can be used for unified epidemiologic studies, for example to assess the effect of a treatment that boosts collagen formation within the patient's body, which is a medical strategy of the future with several drugs currently being developed to stimulate the cells involved in collagen formation.

Finally, using the cutaneous replica makes the microrelief of the skin more visible than directly analysing the skin of the patient, and, as most skin patching device technologies used to form the cutaneous replica are harmless to the health of the patient, and the skin patching device can be used without contraindication.

Apart from the characteristics mentioned above in the previous paragraphs, the method according to a first aspect of the invention may have one or several complementary characteristics among the following characteristics considered individually or in any technically possible combinations.

According to a variant, the method further includes a step of sending the image of the cutaneous replica to a distant server, the step of sending being subsequent to the step of acquisition, and the step of processing, the step of extraction and the step of determination being remotely performed by the distant server.

Thanks to this variant, the step of processing of the acquired image, the step of extraction of the at least one feature and the step of determination of the characteristic length can be centralized on a unique distant server. This further allows lighten implementation of the procedure by a practitioner. Moreover, this allows for a unified processing of many cutaneous replica images from many patients, as it ensures that the method is implemented the same way for each patient. The method is then useful in the case of an epidemiological study. The method also helps practitioners providing homogeneous and unified cares to patients, diminishing ill-suited treatments. Furthermore, the practitioner does not need to possess a dedicated equipment for performing the whole procedure as it is decentralized on a distant server. The practitioner then only requires the skin patching device and the acquisition device, which are not expensive to acquire.

According to another variant, the extracted features are chosen among the following features: the areas of at least part the geometrical shapes, the perimeters of at least part the geometrical shapes, the number of geometrical shapes, or any combination thereof.

Thanks to this variant, the extracted features are representative of the size and the anisotropy of the microrelief of the skin of the patient.

According to some embodiment of the invention, the characteristic length $L_c$ is given by:

$$L_c = \frac{1}{N_b} \sum_{i=1}^{N_b} \left( \sqrt{A_i} + \frac{P_i}{4} \right)$$

, with $N_b$ the number of geometrical shapes, $A_i$ the area of the i-th geometrical shape, and $P_i$ the perimeter of the i-th geometrical shape.

Thanks to this embodiment, the characteristic length is representative of the size and the anisotropy of the microrelief of the skin of the patient.

According to another embodiment, the method further includes a step of determining a collagenic organs quality and/or an actual biological age of the patient by comparing the characteristic length to a characteristic length of reference.

Thanks to this embodiment, the quality of collagenic organs and/or the actual biological age of the patient can be determined from the characteristic length.

According to some embodiment, the step of processing, the step of extraction and the step of determination are carried out by a machine learning predictive algorithm.

This embodiment allows for the simultaneous processing of large amounts of images, therefore allowing for faster unified processing of the images.

According to a second aspect of the invention, it is provided a system for determining a characteristic length representative of a quality of collagenic organs of a patient, comprising:

- an acquisition device adapted to acquire an image of a cutaneous replica of the microrelief of the skin surface of the patient, comprising an optical device adapted to acquire the image of the cutaneous replica;
- a processing system configured to execute the following steps:

  - Processing of said acquired image in order to obtain a processed image, said processed image being formed by a plurality of geometrical shapes;

  - Extraction of a plurality of features from the plurality of geometrical shapes;
  - Determination of the characteristic length representative of the quality of the collagenic organs of the patient based on the extracted features.

The system of the invention allows the execution of the method of the invention with cheap equipment as it only requires few components. The system can thus be implemented in several environment, such as hospital, pharmacy or private practice, without the burden of costly equipment.

In some embodiments, the system further comprises a skin patching device adapted to be applied on a portion of the skin surface of the patient and to obtain the cutaneous replica.

Thanks to this embodiment, the skin patching device allows for a more robust implementation of the method of the invention as the microrelief of the skin is easier to observe on the cutaneous replica. Moreover, the skin patching device can be used without contraindication as most skin patching device technologies are harmless to the health of the patient.

In some embodiments, the acquisition device further comprise a light source adapted to illuminate the cutaneous replica during the acquisition of the image.

Thanks to this embodiment, the illumination of the cutaneous replica can be controlled via the light source, thus reducing variabilities of the acquisition due to the environmental illumination and increasing the robustness of the method of the invention.

In some embodiment, the light source of the acquisition device is an annular light source.

Thanks to this embodiment, the illumination of the cutaneous replica is controlled by a dedicated equipment and avoids environment dependent bias on the acquisition of the image.

In some embodiment, the light source of the acquisition device is a low-angled light source.

Thanks to this embodiment, the light source enables to bring out the microrelief of the skin on the cutaneous replica. The microrelief is then more visible and easier to analyse

In some embodiments, the acquisition device further comprises a receiving support adapted to receive the skin patching device.

The system also allows for a robust implementation of the method of the invention as the receiving support is adapted to receive the skin patching device, therefore lowering measurement errors from the operator.

According to a variant, the optical device resolution is 150 pixels/mm.

Thanks to this variant, the resolution of the optical device is sufficient to precisely image the microrelief in the image as the microrelief characteristic dimension of a skin microrelief is between 150 $\mu$m and 200 $\mu$m.

In some embodiments, the optical device is placed above the light source, the light source is placed above the receiving support, and the optical device, the light source and the receiving support being vertically aligned.

Another aspect of the invention concerns a computer program product comprising instructions which, when the program is executed by a computer, causes the computer to carry out the method according to the invention and any one of the aforementioned embodiments and variants.

The invention and its various applications will be better understood by reading the following description and examining the accompanying figures.

**BRIEF DESCRIPTION OF THE FIGURES**

The figures are presented for information purposes only and in no way limit the invention.

Figure 1 is a flow chart illustrating the steps of the method according to the invention.

Figure 2 schematically represents an embodiment of the system according to the invention.

Figure 3 shows an image a cutaneous replica, an image of a portion of the cutaneous replica and an image of the processed image of the portion of the cutaneous replica obtained by the method of the invention.

Figure 4 shows a comparison between experimental dermis data of measured roughness and elastin to collagen ratios for a plurality of patients.

Figure 5 shows a comparison between experimental dermis data of determined characteristic length obtained by the method of the invention and elastin to collagen ratios for the same plurality of patients used in figure 4.

Figure 6 shows experimental data of measured roughness compared to determined ultimate strain of bone samples using mechanical tests from a plurality of patients.

Figure 7 shows experimental data of determined characteristic lengths from the method of the invention compared to same ultimate strain from the same plurality of patients used in figure 6.

Figure 8 shows experimental data of measured roughness of a plurality of patients from two population categories that are of different age.

Figure 9 shows experimental data of determined characteristic length from the same plurality of patients as in figure 8.

**DETAILED DESCRIPTION**

Devices and methods in accordance with embodiments of the present invention are now described, by way of example and illustration only, and with reference to the accompanying drawings. The description is to be regarded as illustrative in nature and not as restrictive.

Unless explicitly mentioned, a same element appearing on several figures is indicated with the same reference.

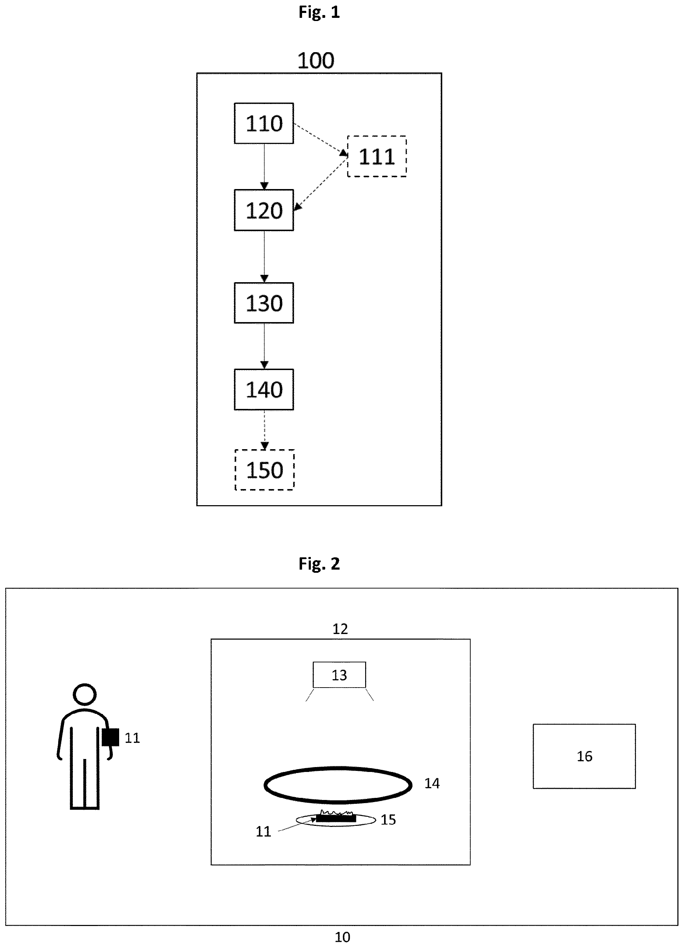

The present invention relates to a method and a system implementing for the method. The method is directed toward the determination of a characteristic length that is representative of the quality of collagenic organs of a patient. The method is divided into at least four main steps that are described on the basis of figure 1. The system comprises one device and a sub-system that are illustrated on figure 2. Two variants of the invention are presented: the first one details the implementation of the method on a local system and the second one details the implementation of the method on a delocalized system. Examples of application are also provided in order to illustrate a possible application.

**Presentation of the method and system according to the invention**

The invention relates to a system and a method implemented by the system for determining a characteristic length representative of a quality of collagenic organs of a patient.

As shown in figure 2, the system 10 comprises:

- an acquisition device 12 comprising:

  - an optical device 13;
  - a light source 12;
  - a receiving support 15;

- a processing system 16.

The system 10 also comprises a skin patching device 11.

As shown in figure 1, the method 100 of the invention comprises a first step 110 of acquisition by the acquisition device 12 of at least one image of a cutaneous replica of the microrelief of a portion of the skin surface of the patient, the cutaneous replica being obtained from the skin patching device 11.

The portion of the skin surface of the patient can be located on any part of the body of the patient. Preferentially, the portion is located on a part of the body that is less exposed to solar illumination which is then more representative of the intrinsic ageing of the patient. For example, the portion of the skin can be located on any one of the two forearms or on the gluteal region of the patient.

The cutaneous replica is formed by applying the skin patching device 11 on the portion of the skin surface of the patient. The cutaneous replica is then a negative of the skin portion microrelief that appears on the surface of the skin patching device 11.

The skin patching device 11 is such that it adapts to the microrelief of the portion of the skin. For example, the skin patching device 11 can be composed of silicone polymer that sinks into the microrelief of the skin portion.

The optical device 13 of the acquisition device 12 is adapted to acquire the image of the cutaneous replica.

The optical device 13 can be any device with a scope that is able to acquire an image of an object. For example, it can be any analogic or numeric photo camera or video camera allowing automatic and immediate transfer to a remote server. Preferentially, the optical device 13 is a device with a scope that is sensitive to the light of the visible spectrum.

In preferred embodiments, the scale factor of the optical device 13 is known or can be determined from the optical device 13 datasheet.

For example, the resolution of the optical device 13 is 150 pixels/mm, and the resolution of the image is 0.7 μm by pixel.

In some embodiments, the resolution of the optical device 13 is small enough to allow downsizing the image without loss of information.

The light source 14 of the acquisition device 12 is adapted to illuminate the cutaneous replica during the acquisition of the image.

The light source 14 can be any type of light source. For example, it can be a white light LED circular light source. The light source 14 can also be directive or non-directive.

Advantageously, the light source 14 is an annular light source.

Advantageously, the light source 14 is a low-angled light source.

The receiving support 15 is a support adapted to the shape of the skin patching device 11.

The receiving support 15 can be any kind of support able to receive the skin patching device 11 and to maintain the skin patching device 11 in a same position. The skin patching device 11 can lay on the receiving support 15 or be attached to it with any kind of attachment (for example, with screws, fixing clips or clamps).

The light source 14 is placed below the optical device 13.

The receiving support 15 is placed below the light source 14, the optical device 13, the light source 14 and the receiving support 15 being vertically aligned.

The image acquired by the acquisition device 12 can be any kind of 2D or 3D image of the cutaneous replica of the portion of the skin of the patient, for example it can be a RGB image or grayscale image coded with a vectorized or non-vectorized format.

One has to note that the image could be directly acquired on the skin portion without using the skin patching device 11 to obtain the cutaneous replica. In such an embodiment, the acquisition device 12 would comprise the optical device 13 and the light source 14 without said receiving support 15.

After acquisition of the image by the acquisition device 12, the image is processed by a processing system 16 of the system 10.

The processing system 16 is configured to execute the following three steps 120, 130 and 140 of the method 100.

A second step 120 of the method 100 is a processing of said acquired image in order to obtain a processed image, said processed image being formed by a plurality of geometrical shapes.

The processed image can be obtained through any kind of image processing algorithm. Preferentially, the image processing algorithm is a segmentation algorithm for segmenting the microrelief of the cutaneous replica image. A pre-processing algorithm can also be implemented before the implementation of the segmentation algorithm. For example, the pre-processing algorithm can be a colour to grayscale conversion algorithm of the image, and the segmentation algorithm can be a watershed algorithm.

The processing of the acquired image creates a processed image formed of a plurality of geometrical shapes.

Preferentially, the processed image is a segmented image formed of a plurality of geometrical shapes obtained after applying a segmentation algorithm to the microrelief of the skin.

The geometrical shape can be any kind of shape that results from a processing algorithm of an image. Preferentially, in the case of a segmentation algorithm, the geometrical shape is a polygon, and the processed image is formed by a plurality of polygons.

A third step 130 of the method 100 is an extraction of at least one feature, preferentially a plurality of features, from the geometrical shapes.

During this step 130, the processed image is analysed by the processing system 16 and at least a feature is extracted from the geometrical shapes. Preferentially, in the case of polygons, several features are extracted from the polygons.

The extraction can produce any kind of features related to the geometrical shape. For example, the at least one feature can be, for a given geometrical shape, the surface, the perimeter, the main orientation, the elongation, the secondary orientation, the circularity, the main axis length, or the secondary axis length or any combination thereof.

In some embodiments, the at least one extracted feature is representative of the size or of the anisotropy of the microrelief.

In some embodiments, the processing system 16 can also determine distribution parameters of the extracted features, such as: mean, median, standard deviation, quartiles, kurtosis, skewness, or any other distribution parameter.

A fourth step 140 of the method 100 is a determination of a characteristic length representative of the quality of the collagenic organs of the patient based on the extracted features.

Based on said at least one feature extracted from each of the geometrical shapes forming the processed image, the processing system 16 determines the characteristic length. The characteristic length is a single scalar.

In some embodiments where the at least one extracted feature is representative of the size or of the anisotropy of the microrelief, the characteristic length is also representative of the size and of the anisotropy of the microrelief.

The feature may be chosen among the following features: the area of at least part of the geometrical shapes, the perimeter of at least part of the geometrical shapes, the number of geometrical shapes, or any combination thereof.

In an advantageous embodiment, the characteristic length $L_c$ is derived using the formula:

$$L_c = \frac{1}{N_b}\sum_{i=1}^{N_b}\left(\sqrt{A_i} + \frac{P_i}{4}\right)$$

, with $N_b$ the number of geometrical shapes, $A_i$ the area of the i-th geometrical shape, and $P_i$ the perimeter of the i-th geometrical shape.

In other embodiments, the determination of the characteristic length is achieved with other formulae and/or based on different features.

The processing system 16 comprises a processor to execute the abovementioned steps 120, 130 and 140.

Advantageously, the method 100 further includes a step 150 of determining a collagenic organs quality and/or an actual biological age of the patient by comparing the characteristic length to a characteristic length of reference. The characteristic length of reference may be given via an abacus or via a scale of values previously measured for reference patients. Reference patients are patients for which the quality of collagenic organs and/or the actual biological age are known.

The comparison of the characteristic value may be any kind of comparison. For example, the characteristic length can be equal, greater than or lesser than the characteristic length of reference.

In an advantageous embodiment, the processing system 16 is embedded in a distant server, not represented.

In that previous embodiment with a remote server, the method 100 further includes a step 111 of sending the image of the cutaneous replica, subsequent to the step 110 of the acquisition of the image, to said remote server. The step 120 of processing, the step 130 of extraction and the step 140 of determination then being remotely performed by the distant server.

In that embodiment, the step 150 for determining the quality of collagenic organs or the actual age of the patient can be also performed by the distant server.

In another embodiment, the processing system 16 may be embedded in the acquisition device 12. The acquisition device 12 and the processing system 16 may also be separated equipment.

The acquisition device 12 is preferably controllable via a user device through a dedicated downloadable application. The user device may be any kind of device used by a user, for example, it can be a mobile phone or a tablet. The user device then comprises instructions that, when executed, causes the acquisition device 12 to carry out the step 110 of the method 100.

In an embodiment, the acquisition device 12 automatically supervises the processing system 16 to execute the steps 120, 130 and 140 of the method 100 to determine the characteristic length, and optionally the step 150.

In a particular embodiment, the step 120 of processing, the step 130 of extraction and the step 140 of determination may be carried out by a machine learning predictive algorithm.

The step 150 for the determination of the quality of the collagenic organs or the actual age of the patient may also be carried out by a machine learning predictive algorithm such as a convolutional neural network.

**First variant of the invention**

In a first variant of the invention, the system 10 is localized at a unique place. For example, the system 10 is used by a practitioner in a private practice for assisting the early detection of osteoporosis.

In such variant, the skin patching device 11 of the system 10 can be a SIFLO™ certified skin patch (Monaderm™, Monaco) composed of a silicon polymer that is nontoxic for the skin and for the mucous membrane. The SIFLO™ structure is light enough to sink inside the skin microrelief.

In an embodiment, several skin patching devices 11 can be placed at different locations on skin of the patient for a more robust analysis throughout the method 100.

In such variant, the acquisition device 12 can be a RaspberryPi single-board computer with an embedded optical device 13, for example, a HQ Raspberry camera with 12.3 MPixels. In some embodiments, the RaspberryPi single-board computer is controlled by a practitioner device, for example a smartphone or a tablet. In some other embodiments, the RaspberryPi single-board computer is autonomous of any practitioner actions.

In some embodiments, the portion of the skin is located on the flexor carpi radiates region, 5 cm distant from the elbow crease. The location of the portion of the skin can variate around this position; for example, it can be $5 \pm 1$ cm from the elbow crease.

The light source 14 can be an annular low-angled white light LED source, placed 15 mm above the cutaneous replica. The internal diameter of the light source 14 can be 11 cm and its external diameter can be 16 cm. The temperature of the light source 14 can be 3500 K.

The skin patching device 11 is placed on the receiving support 15.

The processing system 16 can be another RaspberryPi single-board computer.

After the step 120 of acquisition of the image by the acquisition device 12, the image is provided to the processing system 16.

For example, the acquisition device 12 can automatically send the image to the processing system 16. In this case, the acquisition device 12 and the processing system 16 are mutually connected, for example through a wired or wireless connection.

In other examples, the operator manually transfers the image from the acquisition device 12 to the processing system 16.

During the step 120 of the processing of the image by the processing system 16, the image can be processed using a RGB to grayscale converter. Then the grayscale image can be processed with a H-minima transform and an extended maxima transform before being segmented using a watershed segmentation algorithm.

It is also possible to filter the image, for example with a Gaussian filter, before applying the segmentation algorithm.

Other filters, transforms and algorithms can be used to obtain the processed image.

In some embodiments, the step 120, step 130, and step 140, and optional step 150, are performed on a limited part of the image of the cutaneous replica. For example, the limited part of the image can be a 15x15 mm$^2$ area of the centre on the image.

In some other embodiments, the image can be divided into several sub-images of same or different size and the processing of the image can be performed on one or more of the several sub-images.

After the execution of the step 140 of the method 100, the practitioner can access the characteristic length that is representative of the quality of the collagenic organs of the patient. For example, the practitioner can access this characteristic length on the practitioner device. In such case, the processing system 16 can send the characteristic length via a wired or wireless connection.

The processing system 16 can also perform the step 150 of determination of the quality of the collagenic organs and/or of the actual biological age of the patient.

The whole method 100 can be processed within the time lapse of a medical exam of the patient by the practitioner and the practitioner is able to inform the patient on the quality of his collagenic organs and/or on his actual biological age.

The characteristic length, the determined quality of the collagenic organs or the actual biological age, or any combination thereof, can be available to the patient. For example, the processing system 16 can automatically send these results to a patient device via a wired or wireless connection.

**Second variant of the invention**

In a second advantageous variant of the invention, the processing system 16 is delocalized from the practitioner localisation. For example, the practitioner can have access to the skin patching device 11 and to the acquisition device 12 that are at his working place, while the processing system 16 is localized in another distant place. In such example, the processing system 16 can be a distant processing system (e.g., a server of a medical institution or of a research laboratory) or a cloud service (e.g., a service as a system - SaaS).

In this second variant, the skin patching device 11 and the acquisition device 12 available at the practitioner working place can be the same as in the first variant of the invention.

The main difference with the first variant is that the step 120, the step 130, and the step 140, and the optional step 150, are performed on the distant processing system.

The distant processing system can centralize and handle the steps 120, 130, 140 and 150, for a plurality of practitioners who can be located at different working places and have different medical skills.

The distant processing system can be used to build a database comprising images of cutaneous replica of patient skin and associated determined characteristic length data. The determined qualities of collagenic organs and/or actual biological ages can also be included in the database. For discretion purpose, the database can comprise only anonymous data.

The database can be used to train a machine learning predictive algorithm. This latter can then be implemented in the distant processing system and can be used to carry out the step 120, the step 130, and the step 140, and the optional step 150, of the method 100.

As described in the first variant of the invention, the processing of the image can be performed on a limited part of the image. The image can also be divided into several sub-images and the processing of the image can be performed on one or more of the sub-images.

In this second variant, the acquisition device 12 and the processing system 16 are connected via a wired or wireless connection.

In this second variant, a user device can access the distant processing system via a wired or wireless connection. The access by the user device can be regulated through an access right policy that protects the data on the distant processing system and that ensures that the user has the right to access the distant processing system.

**First example of application**

The proposed example is given as an illustration only and may not be considered as limiting the scope of the invention.

This example deals with a study on the characteristic length determined for a plurality of patients. The study is conducted with several volunteer 60 or more years-old women for applying the aforementioned method 100 and aims to compare the determined characteristic length with data from cutaneous biopsies.

The patients have all a normal body mass index between 18 and 30. They have suffered from a femoral neck fracture but are not subject to chronical pathologies, alcoholism or smoking.

For each patient, a cutaneous replica was formed by applying a skin patching device on one of the forearms. A cutaneous biopsy was also performed on a 5x5 mm$^2$ portion of the gluteal region.

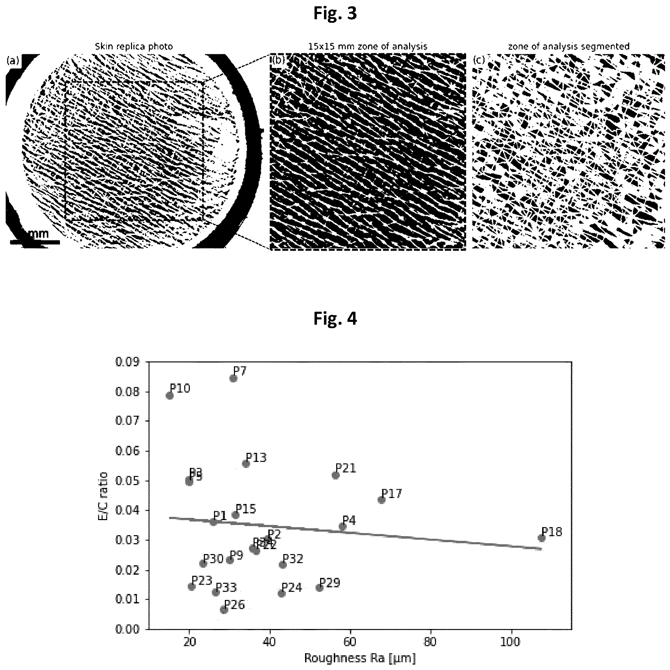

The method according to the invention was applied using the collected cutaneous replica of each patient. For illustrative purpose, an image of one of the cutaneous replicas, an image of a zone of analysis of the cutaneous replica and an image of zone of analysis after applying the segmentation algorithm are presented in figure 3.

The cutaneous replica was also used to measure the roughness of the skin portion using a confocal optic pencil technique.

From the biopsies, elastin to collagen (E/C) ratios were measured using a confocal biphotonic microscope technique.

For each patient, the roughness was compared to the E/C ratio, and the characteristic length was compared to the E/C ratio.

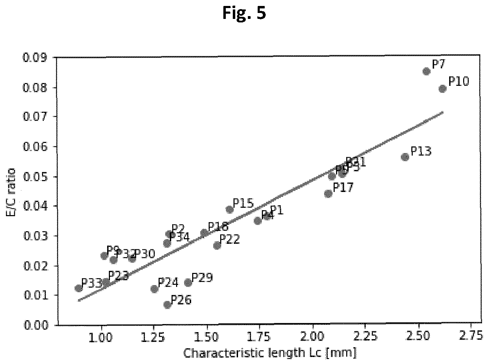

As shown in figure 4, the roughness of the skin microrelief appears to be non-correlated to the E/C ratio, because the cloud of points (noted Px, where x is a coding for patient) representing the distribution of roughness values with respect to the associated E/C ratios does not show a remarkable behaviour. However, as shown in figure 5, the characteristic length shows a strong correlation with the corresponding E/C ratio, because the cloud of points (noted Px, where x is a coding for a patient) representing the distribution of characteristic length values with respect to the associated E/C ratios exhibits a remarkable behaviour.

Based on the conclusion of this study, there is a strong correlation between the quality of collagenic dermis and the characteristic length determined by the method 100 according to the invention.

**Second example of application**

The proposed example is given as an illustration only and may not be considered as limiting the scope of the invention.

This example deals with a study on the characteristic length determined for a plurality of patients. The study is conducted with several volunteer 60 or more years-old women for applying the aforementioned method 100 and to compare the determined characteristic length with data from explanted femoral head.

The patients have all a normal body mass index between 18 and 30. They have suffered from a femoral neck fracture but are not subject to chronical pathologies, alcoholism or smoking.

For each patient, a cutaneous replica was formed by applying a skin patching device on one of the forearms.

The method according to the invention was applied using the collected cutaneous replica of each patient.

The cutaneous replica was also used to measure the roughness of the skin portion using a confocal optic pencil technique.

For each patient, an explanation of the femoral head was also performed. Cylinder-shaped samples were removed from the femoral head thanks to a trephine in a water bath.

Mechanical tests were performed on bone samples to determine mechanical properties of femoral head bone tissues.

Firstly, the women were divided into two groups: a first group with women whose measured roughness is below the roughness median measured over all the women (low_Ra), and a second group group with women whose measured roughness is above the median (high_Ra).

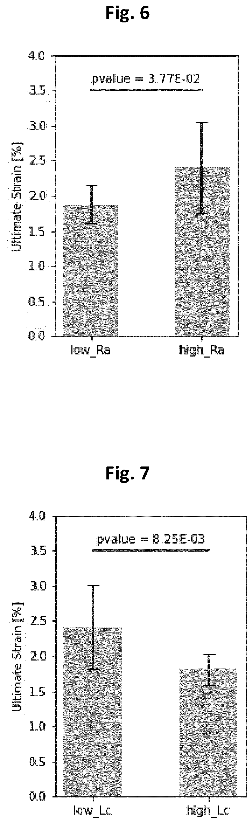

For each group, the ultimate strain of each bone sample is determined and the average value of this strain is represented in the histogram in the figure 6. The standard deviation of the ultimate strain for each group is also plotted.

Secondly, the women were divided into two other groups: one with women whose determined characteristic length is below the median derived over all the women (low_Lc), and the other one with women whose determined characteristic length is above the median (high_Lc).

For each the new groups, the ultimate strain of each bone sample is determined and the average value of this strain is represented in the histogram in the figure 7. The standard deviation of the ultimate strain for each group is also plotted.

From these results, one can conclude that the characteristic length shows a better efficiency at differentiating population groups with low ultimate strain of the bone tissues. Indeed, the ultimate strain is significantly lower for high characteristic lengths associated with a lower quality of the dermis.

**Third example of application**

The proposed example is given as an illustration only and may not be considered as limiting the scope of the invention.

This example deals with a study on the characteristic length determined for a plurality of child and adult patients. The study is conducted with several volunteer 60 or more years-old women and several 13.5 ± 5.5 years-old children. The aim of the study was to apply the aforementioned method 100 and to compare the determined characteristic length determined for both age categories. A comparison of the roughness between the two categories was also conducted.

The adult patients have all a normal body mass index between 18 and 30. They have suffered from a femoral neck fracture but are not subject to chronical pathologies, alcoholism, or smoking.

The child patients have all a normal body mass index between 18 and 28 and are not subject to chronical pathologies, alcoholism, or smoking

For each patient, a cutaneous replica was formed by applying a skin patching device on one of the forearms.

The method according to the invention was applied using the collected cutaneous replica of each patient.

The cutaneous replica was also used to measure the roughness of the skin portion using a confocal optic pencil technique.

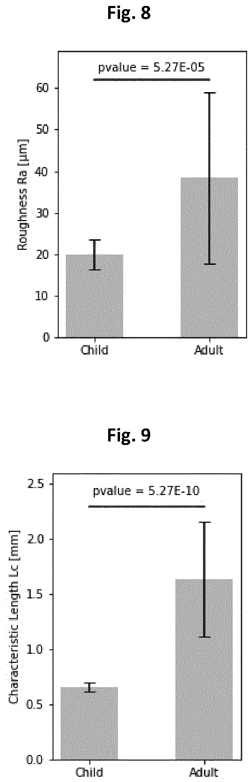

In the figure 8 is presented the mean roughness value of both age groups, along with their respective standard deviation.

In the figure 9 is presented the mean characteristic length value of both age groups, along with their respective standard deviation.

From these results, the characteristic length appears to be representative of the ageing of the skin as on can easily differentiate young and elder patients via the method 100. Moreover, one can conclude that the characteristic length is better to differentiate the two populations than the roughness of the microrelief as the characteristic length is associated to a smaller pvalue.

**Claims**

1. A non-invasive method (100) for determining a characteristic length of skin surface representative of a quality of collagenic organs of a patient, **characterized in that** it comprises the following steps:

   - Acquisition (110) of at least one image of a cutaneous replica of a portion of the skin surface of the patient, the cutaneous replica being obtained from a skin patching device (11) applied on the portion of the skin surface;
   - Processing (120) of said acquired image in order to obtain a processed image, said processed image being formed by a plurality of geometrical shapes;
   - Extraction (130) of a plurality of features from the plurality of geometrical shapes;
   - Determination (140) of the characteristic length representative of the quality of the collagenic organs of the patient based on the extracted features.

2. A method (100) according to claim 1, wherein the method (100) further includes a step (111) of sending the image of the cutaneous replica to a distant server, the step of sending being subsequent to the step (110) of acquisition, and the step (120) of processing, the step (130) of extraction and the step of determination (140) being remotely performed by the distant server.

3. A method (100) according to claim 1 or claim 2, wherein the extracted features are chosen among the following features: the areas of at least part of the geometrical shapes, the perimeters of at least part of the geometrical shapes, the number of geometrical shapes, or any combination thereof.

4. A method (100) according to any of the preceding claims, wherein the characteristic length $L_c$ is given by:

$$L_c = \frac{1}{N_b} \sum_{i=1}^{N_b} \left( \sqrt{A_i} + \frac{P_i}{4} \right)$$

, with $N_b$ the number of geometrical shapes, $A_i$ the area of the i-th geometrical shape, and $P_i$ the perimeter of the i-th geometrical shape.

5. A method (100) according to any of the preceding claims, wherein the method (100) further includes a step (150) of determining a collagenic organs quality and/or an actual biological age of the patient by comparing the characteristic length to a characteristic length of reference.

6. A method (100) according to any of the preceding claims, wherein the step (120) of processing, the step (130) of extraction and the step (140) of determination are carried out by a machine learning predictive algorithm.

7. A system (10) for determining a characteristic length representative of a quality of collagenic organs of a patient, comprising:

   - an acquisition device (12) adapted to acquire an image of a cutaneous replica of the microrelief of the skin surface of the patient, comprising an optical device (13) adapted to acquire the image of the cutaneous replica;
   - a processing system (16) configured to execute the following steps:

     - Processing (120) of said acquired image in order to obtain a processed image, said processed image being formed by a plurality of geometrical shapes;
     - Extraction (130) of a plurality of features from the plurality of geometrical shapes;
     - Determination (140) of the characteristic length representative of the quality of the collagenic organs of the patient based on the extracted features.

8. A system (10) according to claim 7, further comprising a skin patching device (11) adapted to be applied on a portion of the skin surface of the patient and to obtain the cutaneous replica.

9. A system (10) according to claim 7 or claim 8, wherein the acquisition device (12) further comprises a light source (14) adapted to illuminate the cutaneous replica during the step of acquisition (110) of the image.

**10.** A system (10) according to any of claims 7 to 9, wherein the light source (14) of the acquisition device (12) is an annular light source.

**11.** A system (10) according to any of claims 7 to 10, wherein the light source (14) of the acquisition device (12) is a low-angled light source.

**12.** A system (10) according to any of claim 7 to 11, wherein the acquisition device (12) further comprises a receiving support (15) adapted to receive the skin patching device (11).

**13.** A system (10) according to any of claim 7 to 12, wherein the resolution of the optical device (13) is 150 pixels/mm.

**14.** A system (10) according to any of claims 7 to 13, wherein the optical device (13) is placed above the light source (14), the light source (14) is placed above the receiving support (15), and the optical device (13), the light source (14) and the receiving support (15) being vertically aligned.

**15.** A computer program product comprising instructions which, when the program is executed by a computer, causes the computer to carry out the method (100) according to any one of claims 1 to 6.

**Fig. 1**

**Fig. 2**

**Fig. 3**

Skin replica photo     15x15 mm zone of analysis     zone of analysis segmented

**Fig. 4**

**Fig. 5**

**Fig. 6**

**Fig. 7**

**Fig. 8**

**Fig. 9**

**EUROPEAN SEARCH REPORT**

Europäisches Patentamt
European Patent Office
Office européen des brevets

Application Number

EP 21 30 6439

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VÖRÖS E. ET AL: "Age-Related Changes of the Human Skin Surface Microrelief", GERONTOLOGY, vol. 36, no. 5-6, 1 January 1990 (1990-01-01), pages 276-285, XP055902945, CH ISSN: 0304-324X, DOI: 10.1159/000213212 | 1-3,5-9, 11-15 | INV. G06T7/00 |
| Y | * the whole document * | 9-11 | |
| Y,D | WO 2019/149393 A1 (LYON ECOLE CENTRALE [FR]; UNIV PARIS SUD [FR] ET AL.) 8 August 2019 (2019-08-08) | 9-11 | |
| A | * abstract * <br> * figures 1-6 * <br> * page 1 - page 18 * | 1-8, 12-15 | |

**TECHNICAL FIELDS SEARCHED (IPC)**

G06T

The present search report has been drawn up for all claims

| Place of search | Date of completion of the search | Examiner |
|---|---|---|
| Munich | 24 March 2022 | Tessens, Linda |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6439

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2022

| Patent document cited in search report | | Publication date | Patent family member(s) | | Publication date |
|---|---|---|---|---|---|
| WO 2019149393 | A1 | 08-08-2019 | CA 3089918 | A1 | 08-08-2019 |
| | | | EP 3746792 | A1 | 09-12-2020 |
| | | | JP 2021513392 | A | 27-05-2021 |
| | | | US 2021041457 | A1 | 11-02-2021 |
| | | | WO 2019149393 | A1 | 08-08-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019149393 A

**Non-patent literature cited in the description**

- **LIU et al.** Phenotypic Age: a novel signature of mortality and morbidity risk. *bioRxiv, 363291,* 2018

- **SHUSTER.** Osteoporosis, a unitary hypothesis of collagen loss in skin and bone. *Medical Hypotheses,* 2005, vol. 65 (3), 426-432